# EUROPÄISCHE PATENTSCHRIFT

(11) **EP 2 773 273 B1**
(45) Veröffentlichungstag und Bekanntmachung des Hinweises auf die Patenterteilung: **21.10.2015**
(21) Anmeldenummer: 12788477.3
(22) Anmeldetag: 31.10.2012
(51) Int. Cl.: A61B 18/12, A61B 18/14, A61B 18/00

(54) **ELEKTROCHIRURGIEVORRICHTUNG**
ELECTROSURGICAL DEVICE
DISPOSITIF ÉLECTROCHIRURGICAL

(30) Priorität: 31.10.2011 DE 102011085501
(43) Veröffentlichungstag der Anmeldung: 10.09.2014
(73) Patentinhaber: Söring GmbH, 25451 Quickborn (DE)
(72) Erfinder: NEUMANN, Florian, 25451 Quickborn (DE); ZOBAWA, Klaus, 25451 Quickborn (DE)
(74) Vertreter: Glawe, Delfs, Moll
(86) Internationale Anmeldenummer: PCT/EP2012/071592
(87) Internationale Veröffentlichungsnummer: WO 2013/064551

(56) Entgegenhaltungen:
- WO-A1-99/20213
- WO-A2-2008/112931
- US-A1- 2010 130 976

## Beschreibung

Die Erfindung betrifft eine Elektrochirurgievorrichtung, die einen ersten Signalgenerator mit ersten Elektroden und einen zweiten Signalgenerator mit zweiten Elektroden umfasst. Die Signalgeneratoren sind dazu ausgelegt, ein elektrisches Signal zu erzeugen, das über die Elektroden auf einen Patienten übertragen werden kann. So eine Vorrichtung ist bereits aus dem Dokument US2010130976 bekannt.

Die Erfindung betrifft außerdem ein Verfahren zum Betreiben einer solchen Elektrochirurgievorrichtung.

In der Elektrochirurgie wird das Gewebe eines Patienten durch elektrische Energie behandelt, die über Elektroden auf das Gewebe übertragen wird. Der Signalgenerator dient dazu, ein geeignetes elektrisches Signal zu erzeugen. Die Wirkung auf das Gewebe hängt davon ab, welche Energiemenge auf das Gewebe übertragen wird. Beispielsweise kann mittels Elektrochirurgie ein Blutfluss gestillt werden oder Gewebe durchtrennt werden.

Häufig kommen bei Operationen mehrere Elektrochirurgiegeräte gleichzeitig zum Einsatz. So kann etwa mit einem Elektrochirurgiegerät das Gewebe an einer Stelle durchtrennt werden, während andere Elektrochirurgiegeräte gleichzeitig den Blutfluss an anderen Stellen stillen. Bei der Verwendung mehrerer Elektrochirurgiegeräte muss darauf geachtet werden, dass die Gesamtmenge an elektrischer Energie, die durch die Mehrzahl von Elektrochirurgiegeräte in den Körper eingeleitet wird, nicht zu hoch wird. So sollen im Mittel über 1 s nicht mehr als 400 W in den Körper eingeleitet werden.

Bislang muss das OP-Personal die Signalgeneratoren von Hand so einstellen, dass die zulässigen Grenzen eingehalten werden. Es muss also eine Person den Überblick haben, wie viele Elektrochirurgiegeräte aktiv sind und mit welcher Leistung diese betrieben werden. Bei Operationen, bei denen mehrere Personen zusammenarbeiten, ist es nicht ganz einfach, diesen Überblick zu bewahren, Es besteht das Risiko, dass zu viele Elektrochirurgiegeräte parallel zueinander betrieben werden und der Patient mit zu hoher Leistung behandelt wird.

Der Erfindung liegt die Aufgabe zu Grunde, eine Elektrochirurgievorrichtung und ein zugehöriges Verfahren vorzustellen, bei denen das Risiko vermindert ist, dass der Patient mit zu hoher Leistung behandelt wird. Ausgehend vom eingangs genannten Stand der Technik wird die Aufgabe gelöst mit den Merkmalen der unabhängigen Ansprüche. Vorteilhafte Ausführungsformen finden sich in den Unteransprüchen.

Erfindungsgemäß umfasst die Elektrochirurgievorrichtung ein Steuermodul, in dem ein Leistungswert des ersten Signalgenerators und ein Leistungswert des zweiten Signalgenerators zu einem Gesamtleistungswert zusammengeführt werden. Das Steuermodul stellt fest, wenn der Gesamtleistungswert einen vorgegebenen Schwellwert überschreitet.

Die Erfindung hat erkannt, dass es durch die automatische Zusammenführung der Leistungswerte in dem Steuermodul für das OP-Personal leichter wird, die vorgeschriebenen Grenzen einzuhalten. Wenn das Steuermodul eine Überschreitung des vorgegebenen Schwellwerts feststellt, kann auf die Aktivierung weiterer Elektrochirurgiegeräte verzichtet werden bzw. die Leistung in geeigneter Weise reduziert werden.

Die Elektrochirurgievorrichtung kann lediglich zwei Signalgeneratoren umfassen. Die Vorzüge der Erfindung kommen aber in verstärktem Maße zur Geltung, wenn mehr als zwei Signalgeneratoren vorgesehen sind. Je mehr Signalgeneratoren es gibt, desto schwieriger wird es für das OP-Personal, den Überblick zu behalten. Beispielsweise kann mit zwei Signalgeneratoren ein Stück Gewebe abgetrennt werden, während gleichzeitig mit drei weiteren Signalgeneratoren der Blutfluss an verschiedenen Stellen gestillt wird. Das Steuermodul kann in diesem Fall die Leistungswerte aller Signalgeneratoren zu einem Gesamtleistungswert zusammenführen.

Das Steuermodul kann so ausgelegt sein, dass nach Überschreiten des vorgegebenen Schwellwerts ein Signal für das OP-Personal ausgegeben wird. Das Signal kann beispielsweise optisch oder akustisch sein. Das OP-Personal kann dann in geeigneter Weise reagieren und einen oder mehrere Signalgeneratoren außer Betrieb setzen oder die Leistung reduzieren.

Alternativ oder zusätzlich dazu kann das Steuermodul so augelegt sein, dass es nach Überschreiten des vorgegebenen Schwellwerts einen Steuerbefehl ausgibt, der an einen oder mehrere Signalgeneratoren gerichtet ist. Durch den Steuerbefehl kann der Signalgenerator außer Betrieb gesetzt werden. Alternativ kann dem Signalgenerator eine Leistungsgrenze gesetzt werden. Auf diese Weise kann mit dem Steuermodul automatisch sichergestellt werden, dass die an den Patienten abgegebene elektrische Leistung unterhalb vorgegebener Grenzen bleibt.

Die Signalgeneratoren können so ausgelegt sein, dass sie auf unterschiedliche Leistungsstufen eingestellt werden können, wobei jede Leistungsstufe eine unterschiedliche maximale Leistung für den Signalgenerator definiert. Dies bedeutet nicht, dass der Signalgenerator immer mit der in der Leistungsstufe maximal möglichen Leistung betrieben wird, sondern die Leistungsabgabe des Signalgenerators ist innerhalb des durch die maximale Leistung definierten Leistungsbereiches variabel.

Die in dem Steuermodul verarbeiteten Leistungswerte können sich auf die Leistungsstufe der Signalgeneratoren beziehen. Wenn bereits die Summe der maximal möglichen Leistungen unterhalb des Schwellwerts liegt, so ist die Summe der momentan anliegenden tatsächlichen Leistung erst recht kleiner als der Schwellwert. Überschritten wird der Schwellwert bei dieser Ausführungsform, wenn die Summe der Leistungsstufen, also die Summe der innerhalb der Leistungsstufen maximal möglichen Leistungen, oberhalb des Schwellwerts liegt.

Beziehen sich die Leistungswerte allein auf die Leistungsstufe des Signalgenerators, so wird die momentan anliegende tatsächliche Leistung die allermeiste Zeit weit unterhalb des Schwellwerts liegen, da es unwahrscheinlich ist, dass zur gleichen Zeit alle Signalgeneratoren mit der maximalen Leistung der betreffenden Leistungsstufe betrieben werden. Die erfindungsgemäße Elektrochirurgievorrichtung kann deswegen so eingerichtet sein, dass die Leistungswerte sich auf die momentan abgegebene tatsächliche Leistung der Signalgeneratoren beziehen.

Die Elektrochirurgievorrichtung kann so ausgelegt sein, dass permanent die Momentanleistung überwacht wird. Alternativ kann eine zweistufige Vorgehensweise vorgesehen sein, bei der zunächst die Leistungsstufen überwacht werden. Erst wenn die Leistungsstufen in Summe den Schwellwert überschritten haben, wird zu einer Überwachung der Momentanleistungen übergegangen. Dies hat den Vorteil, dass auf der ersten Stufe jeder Signalgenerator mit Sicherheit die Leistung liefern kann, die innerhalb der Leistungsstufe von ihm abgerufen wird. Auf der zweiten Stufe muss damit gerechnet werden, dass zeitweilig einer oder mehrere Signalgeneratoren einer Leistungsbeschränkung unterliegen.

Wenn eine Überschreitung des Schwellwerts festgestellt wird, muss entschieden werden, auf welche Weise die Elektrochirurgievorrichtung so betrieben werden kann, dass die an den Patienten abgegebene Leistung innerhalb der vorgesehenen Grenzen bleibt. Wurde die Überschreitung beispielsweise dadurch verursacht, dass ein weiterer Signalgenerator in Betrieb genommen werden sollte, kann dieser Signalgenerator daran gehindert werden, den Betrieb aufzunehmen. Es bleiben dann die bisherigen Signalgeneratoren in Betrieb, deren Leistungswerte in Summe unterhalb des Schwellwerts liegen. Alternativ kann dem betreffenden Signalgenerator zwar die Aufnahme des Betriebs gestattet werden, ihm aber eine Leistungsgrenze gesetzt werden, die unterhalb der maximalen Leistung der Leistungsstufe liegt.

Bei jeder Überschreitung des Schwellwerts stellt sich die Frage, welchem der Signalgeneratoren eine Leistungsgrenze gesetzt wird bzw. welcher Signalgenerator außer Betrieb gesetzt wird. Eine Reihenfolge kann festgelegt werden, indem den Signalgeneratoren in Form einer Master-Slave-Gestaltung eine Priorität zugeordnet wird. Es können dann die Signalgeneratoren höherer Priorität mit der vollen gewünschten Leistung betrieben werden, während den Signalgeneratoren niedrigerer Priorität eine Leistungsgrenze gesetzt wird, bzw. diese außer Betrieb gesetzt werden.

Zusätzlich oder alternativ dazu kann auch vorgesehen sein, dass eine gegenseitige Überwachung der Signalgeneratoren stattfindet. Es kann dazu eine Mehrzahl von Steuermodulen vorgesehen sein, wobei vorzugsweise jedem Signalgenerator ein Steuermodul zugeordnet ist. In jedem Steuermodul wird der Gesamtleistungswert ermittelt. Dies bietet die Möglichkeit, dass in jedem Signalgenerator vor einer Änderung der Leistung festgestellt wird, ob die Leistungsänderung noch möglich ist, ohne den Schwellwert zu überschreiten. Ist dies nicht der Fall, wird die Leistungsänderung nicht vorgenommen. Es ergibt sich dadurch automatisch, bei welchem der Signalgeneratoren die Leistung beschränkt wird.

Die Erfindung betrifft außerdem ein Verfahren zum Betreiben einer Elektrochirurgievorrichtung, die einen ersten Signalgenerator und einen zweiten Signalgenerator umfasst. Bei dem Verfahren werden ein Leistungswert des ersten Signalgenerators und ein Leistungswert des zweiten Signalgenerators bereitgestellt. Die Leistungswerte werden zu einem Gesamtleistungswert zusammengeführt. Es wird festgestellt, wenn der Gesamtleistungswert einen vorgegebenen Schwellwert überschreitet.

Das Verfahren kann mit weiteren Merkmalen fortgebildet werden, die oben mit Bezug auf die erfindungsgemäße Elektrochirurgievorrichtung beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine erste Ausführungsform einer erfindungsgemäßen Elektrochirurgievorrichtung;
- Fig. 2:: eine zweite Ausführungsform einer erfindungsgemäßen Elektrochirurgievorrichtung;
- Fig. 3:: eine dritte Ausführungsform einer erfindungsgemäßen Elektrochirurgievorrichtung; und
- Fig. 4:: eine der Fig. 2 entsprechende Darstellung, wobei die Vorrichtung für eine bipolare Anwendung ausgelegt ist.

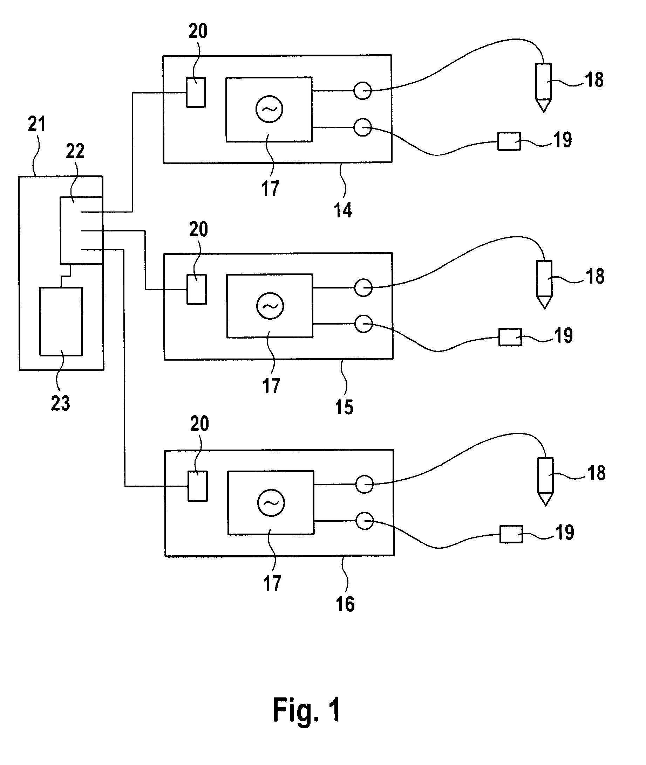

Eine erfindungsgemäße Elektrochirurgievorrichtung in Fig. 1 umfasst ein erstes Elektrochirurgiegerät 14, ein zweites Elektrochirurgiegerät 15 und ein drittes Elektrochirurgiegerät 16. Jedes Elektrochirurgiegerät 14, 15, 16 ist mit einem Signalgenerator 17 ausgestattet, der dazu ausgelegt ist, ein hochfrequentes elektrisches Signal mit einer Frequenz zwischen beilspielsweise 500 kHz und 3000 kHz zu erzeugen. An die Signalgeneratoren sind jeweils eine Aktivelektrode 18 und eine Neutralelektrode 19 angeschlossen.

Die Neutralelektrode 19 wird mit dem Körper des Patienten verbunden. Die Aktivelektrode 18 bildet das Handinstrument, mit dem der Chirurg arbeitet. Wird die Aktivelektrode 18 mit dem Gewebe des Patienten in Berührung gebracht, fließt ein elektrischer Strom durch den Körper des Patienten hindurch zu der Neutralelektrode 19. In unmittelbarer Umgebung der Aktivelektrode 18 hat der elektrische Strom eine erhebliche Wirkung auf das Gewebe, die sich mit zunehmendem Abstand von der Aktivelektrode 18 schnell verliert. Diese lokal begrenzte Wirkung der Aktivelektrode 18 wird dazu genutzt, um beispielsweise das Gewebe zu durchtrennen oder einen Blutfluss zu stillen. Da der Chirurg mit dem Handinstrument lediglich die Aktivelektrode führt, wird diese Anwendung als monopolar bezeichnet.

Durch die Mehrzahl von Elektrochirurgiegeräten 14, 15, 16 wird es möglich, den Patienten gleichzeitig mit einer Mehrzahl von Aktivelektroden 18 zu behandeln, wobei das mit dem Signalgenerator 17 erzeugte elektrische Signal für die einzelnen Aktivelektroden 18 unabhängig voneinander eingestellt und angepasst werden kann. So kann beispielsweise das Elektrochirurgiegerät 14 mit hoher Leistung dazu genutzt werden, um Gewebe zu durchtrennen. Gleichzeitig können die Elektrochirurgiegeräte 15, 16 mit geringerer Leistung betrieben werden, um Blut zu koagulieren. Dabei muss sichergestellt werden, dass die drei Elektrochirurgiegeräte 14, 15, 16 zusammen im Mittel über 1 s betrachtet nicht mehr als 400 W Leistung an den Patienten abgeben. Zulässig wäre es also beispielsweise, das Elektrochirurgiegerät 14 mit einer Leistung von 250 W und gleichzeitig die Elektrochirurgiegeräte 15, 16 mit einer Leistung von 75 W zu betreiben, so dass man in der Summe gerade auf 400 W Leistung kommt. Ein gleichzeitiger Betrieb aller Elektrochirurgiegeräte 14, 15, 16 mit voller Leistung ist hingegen nicht zulässig.

Die Elektrochirurgiegeräte 14, 15, 16 können jeweils auf unterschiedliche Leistungsstufen von beispielsweise 75 W, 150 W und 250 W eingestellt werden, wobei die Leistungsangabe sich auf die Maximalleistung der Leistungsstufe bezieht. Jedes Elektrochirurgiegerät 14, 15, 16 umfasst einen Indikator 20, der die gegenwärtig eingestellte Leistungsstufe über eine Leitung an ein Steuermodul 21 mitteilt. In einem Logikbaustein 22 des Steuermoduls 21 werden die drei von den Elektrochirurgiegeräten 14, 15, 16 mitgeteilten Leistungswerte zu einem Gesamtleistungswert aufaddiert und mit einem in dem Logikbaustein 22 hinterlegten Schwellwert von 400 W verglichen. Überschreitet der Gesamtleistungswert den Schwellwert, wird ein Signal an einen Befehlsgeber 23 gegeben. Der Befehlsgeber 23 übermittelt einen Steuerbefehl an das Elektrochirurgiegerät, von dem die letzte Änderung der Leistungsstufe stammt. Das Elektrochirurgiegerät entnimmt dem Steuerbefehl, dass die zuletzt vorgenommene Änderung der Leistungsstufe nicht möglich ist. Der Betrieb des Elektrochirurgiegeräts wird mit der zuvor eingestellten Leistungsstufe fortgesetzt.

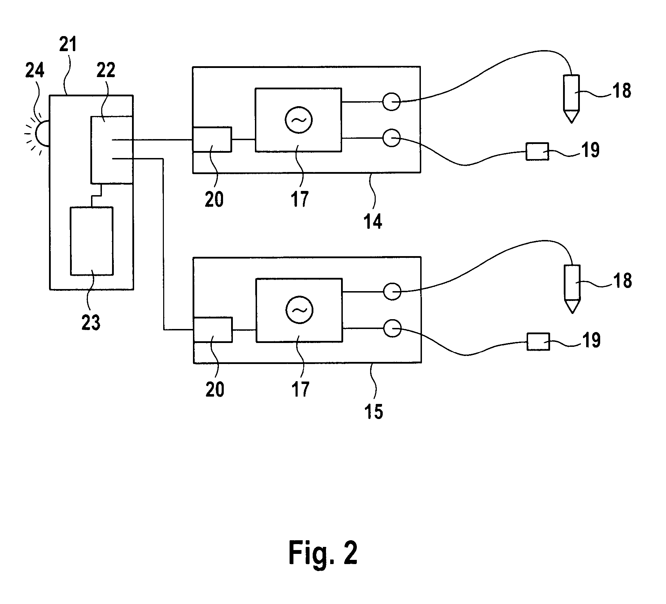

Bei der Ausführungsform gemäß Fig. 2 umfasst die Elektrochirurgievorrichtung zwei Elektrochirurgiegeräte 14, 15. Die Elektrochirurgiegeräte 14, 15 umfassen jeweils einen Signalgenerator 17 zur Erzeugung eines hochfrequenten elektrischen Signals, das über eine Aktivelektrode 18 und eine Neutralelektrode 19 auf das Gewebe eines Patienten übertragen werden kann. Im Unterschied zur Ausführungsform der Fig. 1 teilt der Indikator 20 nicht die Leistungsstufe, sondern die Momentanleistung des Signalgenerators 17 mit. Da die Signalgeneratoren 17 nicht permanent mit der innerhalb der jeweiligen Leistungsstufe möglichen maximalen Leistung betrieben werden, liegt die Momentanleistung regelmäßig niedriger. In dem Logikmodul 22 werden die Leistungswerte der beiden Elektrochirurgiegeräte 14, 15 zu einem Gesamtleistungswert addiert, der sich wie die Momentanleistung laufend ändern kann. Das Logikmodul 22 vergleicht den Gesamtleistungswert lau fend mit dem hinterlegten Schwellwert von 400 W. Wird der Schwellwert überschritten, wird ein Signal an den Befehlsgeber 23 gegeben und eine Warnleuchte 24 aktiviert. Der Befehlsgeber 23 gibt einen Steuerbefehl an eines der Elektrochirurgiegeräte 14, 15, so dass das betreffende Elektrochirurgiegerät die Leistung wieder reduziert. Vorzugsweise ist zu diesem Zweck eines der Elektrochirurgiegeräte 14, 15 als Master und das andere als Slave eingerichtet. Der Steuerbefehl zur Verminderung der Leistung geht an das Slave-Elektrochirurgiegerät. Dadurch ist sichergestellt, dass beim Master-Elektrochirurgiegerät keine überraschende Verminderung der Leistung stattfinden kann.

Alternativ kann die Elektrochirurgievorrichtung auch so eingerichtet sein, dass auf einer ersten Stufe lediglich die Leistungsstufen überwacht werden. Solange die Leistungsstufen in Summe unterhalb des Schwellwerts liegen, kann jeder Signalgenerator jederzeit die Leistung liefern, die innerhalb der Leistungsstufe von ihm abgerufen wird. Überschreiten die Leistungsstufen in Summe den Schwellwert wird zu einer zweiten Stufe übergegangen, bei der die Momentanleistungen der Signalgeneratoren überwacht werden. Auf der zweiten Stufe müssen die Benutzer damit rechnen, dass die Signalgeneratoren die gewünschte Leistung zeitweilig nicht zur Verfügung stellen können, weil sie gerade einer Leistungsbeschränkung unterliegen.

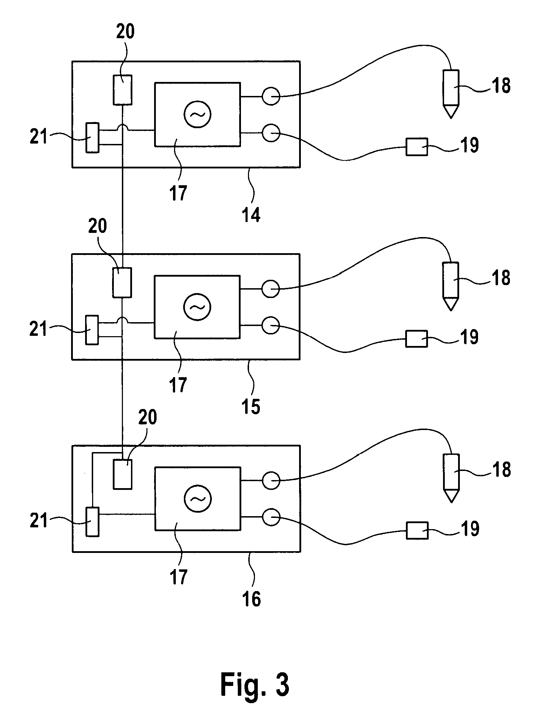

Die Fig. 3 zeigt wieder eine Ausführungsform mit drei Elektrochirurgiegeräten 14, 15, 16. Die Indikatoren 20 der drei Elektrochirurgiegeräte 14, 15, 16 sind miteinander verbunden, so dass der Gesamtleistungswert in jedem der Elektrochirurgiegeräte 14, 15, 16 abgerufen werden kann. Jedes der Elektrochirurgiegeräte umfasst ein Steuermodul 21, in dem der Gesamtleistungswert mit dem Schwellwert verglichen wird. Soll in einem der Elektrochirurgiegeräte die Leistung geändert werden, kann das Steuermodul feststellen, ob durch diese Leistungsänderung der Schwellwert überschritten würde. Ist dies der Fall, kann das betreffende Elektrochirurgiegerät an der Leistungsänderung gehindert werden.

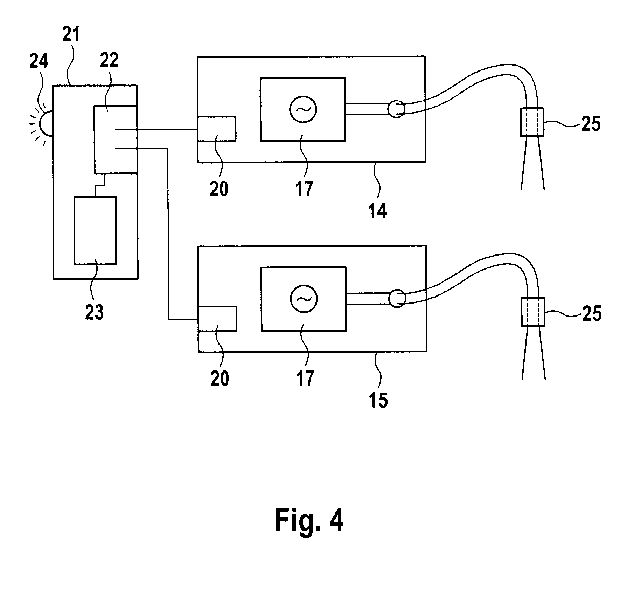

Die Fig. 4 zeigt eine der Fig. 2 entsprechende Ausführungsform, bei der das Handinstrument 25 als Pinzette ausgebildet ist, die beide Elektroden in sich vereint. Wenn der Chirurg das Handinstrument bewegt, führt er immer beide Elektroden zugleich. Die elektrische Energie wirkt in erster Linie auf das zwischen den Elektroden eingeschlossene Gewebe. Diese Art der Anwendung wird als bipolar bezeichnet.

## Patentansprüche

1. Elektrochirurgievorrichtung, umfassend einen ersten Signalgenerator (17) mit ersten Elektroden (18, 19) und einen zweiten Signalgenerator (17) mit zweiten Elektroden (18, 19), wobei die Signalgeneratoren (17) dazu ausgelegt sind, ein elektrisches Signal zu erzeugen, das über die Elektroden (18, 19) auf einen Patienten übertragen werden kann, **dadurch gekennzeichnet, dass** ein Steuermodul (21) vorgesehen ist, in dem ein Leistungswert des ersten Signalgenerators (17) und ein Leistungswert des zweiten Signalgenerators (17) zu einem Gesamtleistungswert zusammengeführt werden, und das feststellt, wenn der Gesamtleistungswert einen vorgegebenen Schwellwert überschreitet.

2. Elektrochirurgievorrichtung nach Anspruch 1, **dadurch gekennzeichnet, dass** mehr als zwei Signalgeneratoren (17) vorgesehen sind und dass das Steuermodul (23) die Leistungswerte der mehr als zwei Signalgeneratoren (17) zu einem Gesamtleistungswert zusammenführt.

3. Elektrochirurgievorrichtung nach Anspruch 1 oder 2, **dadurch gekennzeichnet, dass** das Steuermodul (21) nach dem Überschreiten des vorgegebenen Schwellwerts ein Signal für das OP-Personal ausgibt.

4. Elektrochirurgievorrichtung nach einem der Ansprüche 1 bis 3, **dadurch gekennzeichnet, dass** das Steuermodul (21) nach dem Überschreiten des vorgegebenen Schwellwerts einen Steuerbefehl ausgibt, der an einen oder mehrere Signalgeneratoren (17) gerichtet ist.

5. Elektrochirurgievorrichtung nach Anspruch 4, **dadurch gekennzeichnet, dass** der Signalgenerator (17) durch den Steuerbefehl außer Betrieb gesetzt wird.

6. Elektrochirurgievorrichtung nach Anspruch 4, **dadurch gekennzeichnet, dass** dem Signalgenerator (17) durch den Steuerbefehl eine Leistungsgrenze gesetzt wird.

7. Elektrochirurgievorrichtung nach einem der Ansprüche 1 bis 6, **dadurch gekennzeichnet, dass** das Steuermodul (21) dazu ausgelegt ist, die Leistungsstufen der Signalgeneratoren (17) zu einem Gesamtleistungswert zusammenzuführen.

8. Elektrochirurgievorrichtung nach einem der Ansprüche 1 bis 7, **dadurch gekennzeichnet, dass** das Steuermodul (21) dazu ausgelegt ist, die Momentanleistungen der Signalgeneratoren (17) zu einem Gesamtleistungswert zusammenzuführen.

9. Elektrochirurgievorrichtung nach Anspruch 8, **dadurch gekennzeichnet, dass** das Steuermodul (21) dazu ausgelegt ist, auf einer ersten Stufe die Leistungsstufen der Signalgeneratoren (17) zu einem Gesamtleistungswert zusammenzuführen und auf einer zweiten Stufe die Momentanleistungen der Signalgeneratoren (17) zu einem Gesamtleistungswert zusammenzuführen und dass zur zweiten Stufe gewechselt wird, wenn der Gesamtleistungswert auf der ersten Stufe den Schwellwert überschritten hat.

10. Elektrochirurgievorrichtung nach einem der Ansprüche 1 bis 9, **dadurch gekennzeichnet, dass** den Signalgeneratoren (17) eine Priorität zugeordnet ist, aus der sich ergibt, bei welchem der Signalgeneratoren (17) eine Leistungsbegrenzung durchgeführt wird.

11. Elektrochirurgievorrichtung nach einem der Ansprüche 1 bis 10, **dadurch gekennzeichnet, dass** eine Mehrzahl von Steuermodulen (21) vorgesehen ist und dass jedes Steuermodul (21) einem Signalgenerator (17) zugeordnet ist.

12. Verfahren zum Betreiben einer Elektrochirurgievorrichtung, die einen ersten Signalgenerator (17) mit ersten Elektroden (18, 19) und einen zweiten Signalgenerator (17) mit zweiten Elektroden (18, 19) umfasst, mit folgenden Schritten:
a. Bereitstellen eines Leistungswerts des ersten Signalgenerators (17);
b. Bereitstellen eines Leistungswerts des zweiten Signalgenerators (17);
c. Zusammenführen der Leistungswerte zu einem Gesamtleistungswert; und
d. Feststellen, ob der Gesamtleistungswert einen vorgegebenen Schwellwert überschreitet.

## Claims

1. An electrosurgical device comprising a first signal generator (17) having first electrodes (18, 19) and a second signal generator (17) having second electrodes (18, 19), wherein the signal generators (17) are designed to generate an electrical signal that can be transmitted to a patient via the electrodes (18, 19), **characterized in that** a control module (21) is provided, in which a power value of the first signal generator (17) and a power value of the second signal generator (17) are combined to form a total power value and which ascertains whether the total power value exceeds a predefined threshold value.

2. The electrosurgical device as claimed in claim 1, **characterized in that** more than two signal generators (17) are provided, and **in that** the control module (23) combines the power values of the more than two signal generators (17) to form a total power value.

3. The electrosurgical device as claimed in claim 1 or 2, **characterized in that** the control module (21) outputs a signal for the operation personnel after the predefined threshold value has been exceeded.

4. The electrosurgical device as claimed in any of claims 1 to 3, **characterized in that**, after the predefined threshold value has been exceeded, the control module (21) outputs a control command directed to one or more signal generators (17).

5. The electrosurgical device as claimed in claim 4, **characterized in that** the signal generator (17) is deactivated by the control command.

6. The electrosurgical device as claimed in claim 4, **characterized in that** a power limit is set for the signal generator (17) by the control command.

7. The electrosurgical device as claimed in any of claims 1 to 6, **characterized in that** the control module (21) is designed to combine the power levels of the signal generators (17) to form a total power value.

8. The electrosurgical device as claimed in any of claims 1 to 7, **characterized in that** the control module (21) is designed to combine instantaneous powers of the signal generators (17) to form a total power value.

9. The electrosurgical device as claimed in claim 8, **characterized in that** the control module (21) is designed, at a first stage, to combine the power levels of the signal generators (17) to form a total power value and, at a second stage, to combine the instantaneous powers of the signal generators (17) to form a total power value, and **in that** a change to the second stage is made if the total power value at the first stage has exceeded the threshold value.

10. The electrosurgical device as claimed in any of claims 1 to 9, **characterized in that** the signal generators (17) are assigned a priority that reveals for which of the signal generators (17) a power limitation is carried out.

11. The electrosurgical device as claimed in any of claims 1 to 10, **characterized in that** a plurality of control modules (21) are provided, and **in that** each control module (21) is assigned to a signal generator (17).

12. A method for operating an electrosurgical device comprising a first signal generator (17) having first electrodes (18, 19) and a second signal generator (17) having second electrodes (18, 19), comprising the following steps:
a. providing a power value of the first signal generator (17);
b. providing a power value of the second signal generator (17);
c. combining the power values to form a total power value; and
d. ascertaining whether the total power value exceeds a predefined threshold value.

## Revendications

1. Dispositif de chirurgie électrique comprenant un premier générateur de signaux (17) avec des premières électrodes (18, 19) et un deuxième générateur de signaux (17) avec des deuxièmes électrodes (18, 19), les générateurs de signaux (17) étant conçus pour générer un signal électrique qui peut être transmis par les électrodes (18, 19) à un patient, **caractérisé en ce qu'**un module de commande (21), dans lequel une valeur de puissance du premier générateur de signaux (17) et une valeur de puissance du deuxième générateur de signaux (17) sont regroupées en une valeur de puissance totale, détermine quand la valeur de puissance totale dépasse une valeur seuil prédéterminée.

2. Dispositif de chirurgie électrique selon la revendication 1, **caractérisé en ce que** plus de deux générateurs de signaux (17) sont prévus et **en ce que** le module de commande (23) regroupe les valeurs de puissance des plus de deux générateurs de signaux (17) en une valeur de puissance totale.

3. Dispositif de chirurgie électrique selon la revendication 1 ou 2, **caractérisé en ce que** le module de commande (21) envoie, après le dépassement de la valeur seuil prédéterminée, un signal pour le personnel OP.

4. Dispositif de chirurgie électrique selon l'une des revendications 1 à 3, **caractérisé en ce que** le module de commande (21) envoie, après le dépassement de la valeur seuil prédéterminée, une instruction de commande qui est adressée à un ou plusieurs générateurs de signaux (17).

5. Dispositif de chirurgie électrique selon la revendication 4, **caractérisé en ce que** le générateur de signaux (17) est mis hors service par l'instruction de commande.

6. Dispositif de chirurgie électrique selon la revendication 4, **caractérisé en ce qu'**une limite de puissance est imposée au générateur de signaux (17) par l'instruction de commande.

7. Dispositif de chirurgie électrique selon l'une des revendications 1 à 6, **caractérisé en ce que** le module de commande (21) est conçu pour regrouper les étages de puissance des générateurs de signaux (17) en un valeur de puissance totale.

8. Dispositif de chirurgie électrique selon l'une des revendications 1 à 7, **caractérisé en ce que** le module de commande (21) est conçu pour regrouper les puissances momentanées des générateurs de signaux (17) en une valeur de puissance totale.

9. Dispositif de chirurgie électrique selon la revendication 8, **caractérisé en ce que** le module de commande (21) est conçu pour regrouper, sur un premier étage, les étages de puissance des générateurs de signaux (17) en une valeur de puissance totale et, sur un deuxième étage, les puissances momentanées des générateurs de signaux (17) en une valeur de puissance totale et **en ce qu'**on passe au deuxième étage lorsque la valeur de puissance totale a dépassé la valeur seuil sur le premier étage.

10. Dispositif de chirurgie électrique selon l'une des revendications 1 à 9, **caractérisé en ce qu'**une priorité est accordée aux générateurs de signaux (17), qui indique dans lequel les générateurs de signaux (17) une limitation de puissance est imposée.

11. Dispositif de chirurgie électrique selon l'une des revendications 1 à 10, **caractérisé en ce qu'**une pluralité de modules de commande (21) est prévue et **en ce que** chaque module de commande (21) correspond à un générateur de signaux (17).

12. Procédé d'utilisation d'un dispositif de chirurgie électrique qui comprend un premier générateur de signaux (17) avec des premières électrodes (18, 19) et un deuxième générateur de signaux (17) avec des deuxièmes électrodes (18, 19), avec les étapes suivantes :
a. mise à disposition d'une valeur de puissance du premier générateur de signaux (17) ;
b. mise à disposition d'une valeur de puissance du deuxième générateur de signaux (17) ;
c. regroupement des valeurs de puissance en une valeur de puissance totale ; et
d. détermination si la valeur de puissance totale dépasse une valeur seuil prédéterminée.
